# EUROPEAN PATENT SPECIFICATION

(11) **EP 3 727 352 B1**
(45) Date of publication and mention of the grant of the patent: **21.08.2024**
(21) Application number: 18829502.6
(22) Date of filing: 12.12.2018
(51) Int. Cl.: A61K 9/70, A61K 9/00, A61K 47/10, A61K 47/36, A61K 31/46

(54) **SHEET FORMULATION FOR ORAL USE**
BLATTFORMULIERUNG ZUR ORALEN ANWENDUNG
FORMULATION DE FEUILLE À USAGE ORAL

(30) Priority: 18.12.2017 JP 2017241321
(43) Date of publication of application: 28.10.2020
(73) Proprietor: SSP Co., Ltd., Japan, Shinjuku-ku Tokyo 163-1488 (JP)
(72) Inventor: OSAWA Mizuho, Tokyo 163-1488 (JP); YOSHIMURA Satoru, Tokyo 163-1488 (JP)
(74) Representative: Santarelli
(86) International application number: PCT/JP2018/045639
(87) International publication number: WO 2019/124184

(56) References cited:
- EP-A1- 2 161 021
- EP-A1- 2 233 134
- EP-A2- 2 431 028
- WO-A1-2012/083269
- JP-A- 2002 255 797
- YOGYATA S PATHARE ET AL: "Polymers used for Fast Disintegrating Oral Films: A Review", INTERNATIONAL JOURNAL OF PHARMACEUTICAL SCIENCES REVIEW AND RESEARCH, 1 January 2013 (2013-01-01), pages 169 - 178, XP055136062, Retrieved from the Internet <URL:http://globalresearchonline.net/journalcontents/v21-1/29.pdf> [retrieved on 20140822]
- MUHAMMAD MAHBOOB ET AL: "Oral Films: A Comprehensive Review", INTERNATIONAL CURRENT PHARMACEUTICAL JOURNAL, 18 November 2016 (2016-11-18), pages 111 - 117, XP055558796, Retrieved from the Internet <URL:http://www.icpjonline.com/documents/Vol5Issue12/03.pdf> DOI: 10.3329/icpj.v5i12.30413

## Description

### [Technical Field]

The present invention relates to a sheet formulation for oral use; more specifically, it relates to a sheet formulation for oral use in which the bitter taste of scopolamine butylbromide is masked, and which has flexibility.

### [Background Art]

Recently there has been increasing demand for formulations that are easy to administer orally even in elderly people with decreased physiological function, for example, and in patients with dysphagia. For example, sheet formulations for oral use, which are mostly in thin film form, that dissolve or disintegrate in saliva when placed on the tongue and can be ingested without water are being developed.

Sometimes, strongly bitter tasting components are incorporated as drugs into these sheet formulations for oral use. For example, sheet formulations for oral use into which scopolamine butylbromide has been incorporated are problematic in that, when ingested they taste very strongly bitter, and willingness to ingest the formulation decreases.

Technology whereby a combination of stevioside sweetener and highly sweet sweetener is incorporated has already been reported as a method for preparing a sheet formulation in which the bitter taste of an unpleasant-tasting drug such as scopolamine butylbromide is concealed (cited patent 1). However, such sheet formulation for oral use is disadvantageous in that pullulan is used as the base, and so it has low flexibility and cracks readily when stored in over-dry conditions such as extreme low-humidity environment. There is therefore demand for a sheet formulation of excellent flexibility that does not crack even when stored in over-dry conditions.

### [Citation List]

### [Patent Literature]

[PTL 1] Japanese patent number 5539503. PTL 1 discloses in Table 14 a quickly soluble oral film dosage for effectively masking an unpleasant taste of butylscopolamine, wherein the composition comprises inter alia sucralose, acesulfame potassium, menthol and butylscopolamine.

### [Summary of Invention]

### [Technical Problem]

Therefore, the problem to be solved by the invention is the provision of a sheet formulation for oral use that decreases the bitter taste of scopolamine butylbromide and has excellent flexibility, and has an ability of preventing crack formation under over-dry conditions.

### [Solution to problem]

The present inventors conducted diligent research into the development of a sheet formulation for oral use that decreases the bitter taste of scopolamine butylbromide, has a soft and pleasant texture on the tongue, and poses no quality problems; they perfected the present invention upon discovering that by using cellulose ether polymer as the sheet formulation base, and incorporating a specific combination of sweetener, selected from sucralose and/or saccharin sodium, and menthol into the scopolamine butylbromide, it is possible to obtain a sheet formulation for oral use in which the unpleasant bitter taste of scopolamine butylbromide is masked, and which has good flexibility and does not crack readily under over-dry condition.

Specifically, the present invention is a sheet formulation for oral use, comprising a sheet formulation base and components (A) to (C) below:
(A)Scopolamine butylbromide;
(B)Sucralose and/or saccharin sodium ; and (C)Menthol, wherein the sheet formulation base consists of a cellulose ether polymer and wherein the sheet formulation contains 10-950 of cellulose polymer.

### [Advantageous Effects of Invention]

The inventive sheet formulation for oral use is of high practical value because the intense bitter taste caused by scopolamine butylbromide is decreased by the incorporation of a combination of sucralose and/or saccharin sodium, and menthol. Furthermore, its flexibility is increased by the use of cellulose polymer as base of sheet.

### [Description of Embodiments]

The inventive sheet formulation for oral use contains scopolamine butylbromide as active ingredient, and in order to mask the bitterness of the scopolamine butylbromide, a combination of sucralose and/or saccharin sodium, and menthol, is incorporated. Also, cellulose ether polymer is used as base of the sheet, wherein the sheet formulation contains 10-95% of cellulose polymer.

Scopolamine butylbromide (hereafter referred to as "component (A)"), which is the active ingredient in the inventive sheet formulation for oral use (hereafter referred to as "the inventive sheet formulation"), is a widely known drug that effectively relieves stomachache, abdominal pain and the pain of stomach cramps by easing excess gastrointestinal tension and convulsions; its molecular formula is C₂₁H₃₀BrNO₄ and its molecular weight is 440.37.

There are no particular limitations on the amount of component (A) contained in the inventive sheet formulation; for example, the formulation contains preferably 1-50% by mass (hereafter referred to as simply "%"), more preferably 2-35%, and even more preferably 20-30%.

As for the sucralose and/or saccharin sodium (hereafter referred to as "component (B)") in the inventive sheet formulation, both of these components are known as sweeteners. There are no particular limitations on the amount of component (B) contained in the inventive sheet formulation. For example, the formulation contains preferably around 0.1-30%, more preferably 0.3-20%, and even more preferably 0.5-10%. Most preferably it contains 2-5%.

Moreover, the component (C) menthol (hereafter referred to as "component (C)") in the inventive sheet formulation is a compound that is commonly known as a cold-feeling material, stabilizer, flavoring agent, refreshing agent, etc. Examples of component (C) include DL-menthol and L-menthol, and of these, L-menthol is preferred. There are no particular limitations on the amount of component (C) contained in the inventive sheet formulation. For example, the formulation contains preferably 0.1-40%, more preferably 0.2-30%, and even more preferably 0.5-30%.

Note that when sucralose is used as abovementioned component (B) in the inventive sheet formulation, there are no particular limitations on the mass ratio with component (C); for example, there are preferably 0.0025-300 parts by mass, more preferably 0.01-100 parts by mass, and even more preferably 0.1-20 parts by mass of component (C) per 1 part by mass of sucralose.

Also, when saccharin sodium is used as abovementioned component (B) in the inventive sheet formulation, there are no particular limitations on the mass ratio with component (C); for example, there are preferably 0.0025-300 parts by mass, more preferably 0.01-100 parts by mass, and even more preferably 0.1-20 parts by mass of component (C) per 1 part by mass of saccharin sodium.

The inventive sheet formulation contains one cellulose ether polymer or a plurality of cellulose ether polymers as its base. Examples of these cellulose ether polymers can include cellulose ethers in which some of the hydroxyl groups on the cellulose have been substituted by methyl groups, ethyl groups, hydroxyethyl groups, hydroxypropyl groups, carboxyl groups or the like.

There are no particular limitations with regard to specific examples of the cellulose ether polymers to be used, and examples include methyl cellulose, ethyl cellulose, hydroxyethyl cellulose, hydroxypropyl cellulose, hydroxypropyl methylcellulose and carboxymethyl cellulose. Of these, hydroxypropyl cellulose and hypromellose are preferred.

The amount of above-mentioned cellulose ether polymer contained in the inventive sheet formulation, is preferably around 10-95%, more preferably 10-75%, and even more preferably 10-50%. Most preferably, it contains 30-45%.

The inventive sheet formulation can be produced by a known method. For example, it can be produced by adding abovementioned components (A) to (C) into a solution obtained by dissolving or suspending the cellulose ether polymer that is to be the base, and then moulding the resulting solution to obtain a sheet.

More specifically, for example, components (A) to (C), cellulose ether polymer drug and the necessary optional components are added into a suitable amount of purified water, lower alcohol or mixture thereof, and dissolved or dispersed by stirring to prepare a drug-containing solution. The prepared drug-containing solution is then coated onto a release film of polyethylene phthalate or the like, and dried to obtain a sheet formulation.

When preparing the inventive sheet formulation, as described above, optional components can be added, provided that the effects of the present invention are not impaired. Examples of such optional components include sweeteners other than component (B), acidulants, plasticizers, excipients, emulsifiers, colorants and fragrances.

Examples of the abovementioned sweeteners other than component (B) include reduced maltose mizuame, powdered reduced maltose mizuame, fructose, sucrose, maltose, lactose, saccharose, glucose, maltitol, erythritol, xylitol, mannitol, lactitol, trehalose, acesulphame potassium, aspartame, thaumatin, disodium glycyrrhizinate, trisodium glycyrrhizinate and dipotassium glycyrrhizinate.

Examples of acidulants include citric acid, malic acid, tartaric acid, lactic acid, ascorbic acid, erythorbic acid, succinic acid and fumaric acid.

Moreover, examples of the abovementioned plasticizers include polyethylene glycol (Macrogol 400, Macrogol 1500, etc.), propylene glycol and glycerin; and examples of excipients include titanium oxide, aluminium magnesium hydroxide, magnesium hydroxide, aluminium silicate, silicon dioxide, anhydrous sodium sulphate, anhydrous calcium hydrogen phosphate, sodium chloride, amorphous hydrous silicic acid, magnesium silicate aluminate, calcium silicate, magnesium silicate, light anhydrous silicic acid, heavy anhydrous silicic acid, magnesium oxide, calcium chloride, calcium sulphate, calcium monohydrogen phosphate, calcium hydrogen phosphate, sodium hydrogen phosphate, potassium dihydrogen phosphate, calcium dihydrogen phosphate and sodium dihydrogen phosphate.

Furthermore, examples of the abovementioned emulsifiers include lecithin, soybean lecithin, purified soybean lecithin, purified egg yolk lecithin, sodium lauryl sulphate, glycerin fatty acid esters, sucrose fatty acid esters, sorbitan fatty acid esters, polyoxyethylene hydrogenated castor oil, polyoxyethylene sorbitan fatty acid esters and polyethylene glycol fatty acid esters; and examples of colorants include edible dyes, edible lake dyes, iron sesquioxide and yellow ferric oxide.

The sheet formulation prepared as described above can be a single layer, or it can be a multilayer formulation obtained by laminating several single layers. From a production viewpoint, it is preferably a single layer.

There are no particular limitations on the thickness of the inventive sheet formulation. It is preferably 20-500 um, more preferably 30-300 um. Moreover, there are no particular limitations on the size of the inventive sheet formulation, and it is preferably 0.5-12 cm², more preferably 1.0-10 cm². There are no particular limitations on the shape, provided that it is easy to ingest; for example, rectangle, circle, ellipse or the like can be chosen as the shape, as appropriate.

As an over-the-counter drug, the formulation preferably contains around 10 mg scopolamine butylbromide per dose, to be administered orally no more than 3 times a day. As a drug used in medical treatment, however, 10-20 mg scopolamine butylbromide can be administered per dose, preferably administered orally around 3-5 times a day. Needless to say, the dose can be increased or decreased depending on the age and the condition of the patient.

Thus, the resulting inventive sheet formulation is such that the bitterness of scopolamine butylbromide is masked, and it has a soft and pleasant texture on the tongue. Another characteristic feature is that it is flexible and therefore unlikely to crack.

### [Examples]

The present invention is described in more detail below by means of examples and comparative examples, but the present invention is in no way limited to these.

### Example 1

Inventive film formulation products 1-6 and comparative film formulation products 1 and 2 were prepared as prescribed in Table 1 below. Specifically, 10.0 g scopolamine butylbromide, 2.0 g Macrogol 400, 6.0 g powdered reduced maltose mizuame and 1.0 g sucralose or saccharin sodium were added to 40.0 g weighed water, and dissolved therein. Next, 35.0 g pharmacopoeia ethanol were added to the resulting liquid, then 1.0 g titanium oxide was added and dispersed thoroughly, and then 7.5 g Hypromellose TC-5R and 7.0 g hydroxypropyl cellulose HPC-SSL were added and dissolved. To this solution were added 25.0 g pharmacopoeia ethanol, or a solution obtained by adding the necessary amount of L-menthol to 25.0 g pharmacopoeia ethanol and dissolving, to obtain a drug-containing prepared solution.

A sheet formulation for oral use was produced by uniformly coating a prescribed amount of the abovementioned prepared solution onto a PET film to form a drug-containing layer, and then drying in warm air (at 70°C for 2 hours) . Inventive sheet formulation for oral use products 1-6 and comparative sheet formulation for oral use products 1 and 2 were obtained in this manner. Ten testers used the resulting sheet formulations for oral use, and 30 seconds after ingestion they performed sensory tests for bitterness in accordance with the evaluation criteria below. The scores were averaged and the results are shown in Table 1.

**[Table 1]**

| (mg) | | | | | | | | |
|---|---|---|---|---|---|---|---|---|
| | Inventive product 1 | Inventive product 2 | Inventive product 3 | Inventive product 4 | Inventive product 5 | Inventive product 6 | Comparative product 1 | Comparative product 2 |
| Scopolamine butylbromide | 10.0 | 10.0 | 10.0 | 10.0 | 10.0 | 10.0 | 10.0 | 10.0 |
| Hypromellose TC-5R^{*1} | 7.5 | 7.5 | 7.5 | 7.5 | 7.5 | 7.5 | 7.5 | 7.5 |
| Hydroxypropyl cellulose HPC-SSL^{*2} | 7.0 | 7.0 | 7.0 | 7.0 | 7.0 | 7.0 | 7.0 | 7.0 |
| Titanium oxide | 1.0 | 1.0 | 1.0 | 1.0 | 1.0 | 1.0 | 1.0 | 1.0 |
| Macrogol 400 | 2.0 | 2.0 | 2.0 | 2.0 | 2.0 | 2.0 | 2.0 | 2.0 |
| Powdered reduced maltose mizuame | 6.0 | 6.0 | 6.0 | 6.0 | 6.0 | 6.0 | 6.0 | 6.0 |
| Sucralose | 1.0 | 1.0 | 1.0 | | | | 1.0 | |
| Saccharin sodium | | | | 1.0 | 1.0 | 1.0 | | 1.0 |
| L-menthol | 0.5 | 2.5 | 10.0 | 0.5 | 2.5 | 10.0 | | |
| Total | 35.0 | 37.0 | 44.5 | 35.0 | 37.0 | 44.5 | 34.5 | 34.5 |
| Mean evaluation | 3.3 | 3.1 | 3.4 | 3.4 | 3.6 | 3.1 | 4.0 | 3.8 |

| | | | | | | | | |
|---|---|---|---|---|---|---|---|---|
| *1 Hypromellose TC-5R (manufactured by Shin-Etsu Chemical Co., Ltd.) *2 Hydroxypropyl cellulose HPC-SSL (manufactured by Nippon Soda Co., Ltd.) | | | | | | | | |

### <Evaluation criteria>

Score: Bitterness
5: Unacceptably unpleasant
4: Somewhat unpleasant
3: Cannot say either way
2: Somewhat noticeable but not objectionable
1: Not noticeable at all

From the results for inventive products 1-3 it was confirmed that the bitterness of scopolamine butylbromide is masked by incorporating sucralose and L-menthol. Also, from the results for inventive products 4-6 it was confirmed that the bitterness of scopolamine butylbromide is masked by incorporating saccharin sodium and L-menthol.
* Bitterness score without masking substance was 4.8.

However, the bitterness of scopolamine butylbromide was not masked in comparative example 1 or comparative example 2, which contained sucralose or saccharin sodium but had no L-menthol incorporated.

### Example 2

### Flexibility test

The inventive products 1-6 obtained in Example 1 were dried at 60°C for 12 hours as model over-dry condition, and then 1 cm x 2 cm test samples of thickness 100-200 um were prepared. The test samples were then returned to room temperature and bent 180° using a finger, to confirm flexibility. The results showed that the formulations obtained using cellulose polymer had retained sufficient flexibility not to crack even when dried out at 60°C for 12 hours. In comparison, the formulations (comparative examples) prepared using the watersoluble polysaccharide pullulan instead of cellulose polymer lacked flexibility to the extent that they cracked even on slight impact.

### [Industrial Applicability]

In the inventive sheet formulation, the bitter taste of scopolamine butylbromide is masked such that the inventive sheet formulation can be ingested without water; the inventive sheet formulation is therefore suitable for use in the treatment of the elderly and patients with dysphagia, for example. The inventive sheet formulation is also highly flexible and is therefore of high commercial value because it has an easiness to take and is unlikely to crack even when stored under very dry conditions.

## Claims

1. Sheet formulation for oral use, comprising a sheet formulation base and components (A) to (C) below:
(A) Scopolamine butylbromide;
(B) Sucralose and/or saccharin sodium; and
(C) Menthol,
wherein the sheet formulation base consists of a cellulose ether polymer and wherein the sheet formulation contains 10-950 of cellulose polymer.

2. Sheet formulation according to Claim 1, wherein said cellulose polymer is selected from the group consisting of cellulose ethers in which some of the hydroxyl groups on the cellulose have been substituted by methyl groups, ethyl groups, hydroxyethyl groups, hydroxypropyl groups or carboxyl groups.

3. Sheet formulation according to Claim 1 or 2, wherein the cellulose polymers are (i) hydroxypropyl cellulose, (ii) hypromellose, or (iii) hypromellose and hydroxypropyl cellulose.

4. Sheet formulation according to any of Claim 1 to 3, which contains 1-50% by mass component (A).

5. Sheet formulation according to any of Claims 1 to 4, which contains 0.1-30% by mass component (B).

6. Sheet formulation according to any of Claims 1 to 5, which contains 0.1-40% by mass component (C).

7. Sheet formulation according to any of Claims 1 to 6, wherein component (B) is sucralose, and the mass ratio of said sucralose to component (C) is 1 :0.0025-300 parts by mass.

8. Sheet formulation according to any of Claims 1 to 6, wherein component (B) is saccharin sodium, and the mass ratio of said saccharin sodium to component (C) is
1 :0.0025-300 parts by mass.

## Patentansprüche

1. Blattformulierung zur oralen Verwendung, die eine Blattformulierungsbasis und die nachstehenden Komponenten (A) bis (C) umfasst:
(A) Scopolaminbutylbromid;
(B) Sucralose und/oder Natriumsaccharin; und
(C) Menthol,
wobei die Blattformulierungsbasis aus einem Celluloseetherpolymer besteht und wobei die Blattformulierung 10-95 % Cellulosepolymer enthält.

2. Blattformulierung nach Anspruch 1, wobei das Cellulosepolymer ausgewählt ist aus der Gruppe bestehend aus Celluloseethern, in denen einige der Hydroxylgruppen an der Cellulose durch Methylgruppen, Ethylgruppen, Hydroxyethylgruppen, Hydroxypropylgruppen oder Carboxylgruppen substituiert wurden.

3. Blattformulierung nach Anspruch 1 oder 2, wobei die Cellulosepolymere (i) Hydroxypropylcellulose, (ii) Hypromellose oder (iii) Hypromellose und Hydroxypropylcellulose sind.

4. Blattformulierung nach einem von Anspruch 1 bis 3, die 1-50 Masse-% Komponente (A) enthält.

5. Blattformulierung nach einem der Ansprüche 1 bis 4, die 0,1-30 Masse-% Komponente (B) enthält.

6. Blattformulierung nach einem der Ansprüche 1 bis 5, die 0,1-40 Masse-% Komponente (C) enthält.

7. Blattformulierung nach einem der Ansprüche 1 bis 6, wobei Komponente (B) Sucralose ist und das Massenverhältnis der Sucralose zu Komponente (C) 1:0,0025-300 Massenteile beträgt.

8. Blattformulierung nach einem der Ansprüche 1 bis 6, wobei Komponente (B) Natriumsaccharin ist und das Massenverhältnis des Natriumsaccharins zu Komponente (C) 1:0,0025-300 Massenteile beträgt.

## Revendications

1. Formulation en feuille pour usage oral, comprenant une formulation en feuille de base et les composants (A) à (C) ci-dessous :
(A) butylbromure de scopolamine ;
(B) sucralose et/ou saccharine sodique ; et
(C) menthol,
dans laquelle la formulation en feuille de base consiste en un polymère d'éther de cellulose et dans laquelle la formulation en feuille contient 10-95 % de polymère cellulosique.

2. Formulation en feuille selon la revendication 1, dans laquelle ledit polymère cellulosique est sélectionné dans le groupe consistant en les éthers de cellulose dans lesquels certains des groupes hydroxyles sur la cellulose ont été substitués par des groupes méthyles, des groupes éthyles, des groupes hydroxyéthyles, des groupes hydroxypropyles ou des groupes carboxyles.

3. Formulation en feuille selon la revendication 1 ou 2, dans laquelle les polymères cellulosiques sont (i) l'hydroxypropylcellulose, (ii) l'hypromellose, ou (iii) l'hypromellose et l'hydroxypropylcellulose.

4. Formulation en feuille selon l'une des revendications 1 à 3, qui contient 1-50 % en masse du composant (A).

5. Formulation en feuille selon l'une des revendications 1 à 4, qui contient 0,1-30 % en masse du composant (B).

6. Formulation en feuille selon l'une des revendications 1 à 5, qui contient 0,1-40 % en masse du composant (C).

7. Formulation de feuille selon l'une des revendications 1 à 6, dans laquelle le composant (B) est le sucralose, et le rapport massique dudit sucralose au composant (C) est 1:0,0025-300 parties en masse.

8. Formulation en feuille selon l'une des revendications 1 à 6, dans laquelle le composant (B) est le sodium de saccharine, et le rapport massique dudit sodium de saccharine au composant (C) est 1:0,0025-300 parties en masse.
